# EUROPEAN PATENT APPLICATION

(11) **EP 4 099 259 A1**
(43) Date of publication of application: **07.12.2022**
(21) Application number: 21768502.3
(22) Date of filing: 12.03.2021
(51) Int. Cl.: G06Q 50/02, A01G 7/00

(54) **CULTIVATION SUPPORT SYSTEM, CULTIVATION SUPPORT DEVICE, CULTIVATION SUPPORT METHOD, AND PROGRAM**

(30) Priority: 13.03.2020 JP 2020043700
(71) Applicant: OMRON Corporation, Kyoto-shi, Kyoto 600-8530 (JP); Organic Nico Co., Ltd., Kyoto-shi, Kyoto 610-1132 (JP)
(72) Inventor: HOSOMI, Shinichi, Kyoto-shi, Kyoto 600-8530 (JP); SASAKI, Sho, Kyoto-shi, Kyoto 600-8530 (JP); MIYAJI, Takaaki, Kyoto-shi, Kyoto 600-8530 (JP); NAKAMURA, Arata, Kyoto-shi, Kyoto 610-1132 (JP)
(74) Representative: Isarpatent
(86) International application number: PCT/JP2021/010086
(87) International publication number: WO 2021/182616

(57) **Abstract**

A cultivation support system for supporting cultivation of a plant includes a growth information acquisition unit configured to acquire information on the growing condition of the plant, a growing condition evaluation unit configured to evaluate the growing condition of the plant on a scale of levels, a work content determination unit configured to determine optimum cultivation work for the growing condition of the plant, a work instruction information creation unit configured to create work instruction information indicating work to be performed by a person engaged in the cultivation of the plant, based on the cultivation work determined by the work content determination unit, and an output unit configured to output the work instruction information created by the work instruction information creation unit.

## Description

### TECHNICAL FIELD

The present invention relates to a technique for cultivating plants such as crops, and more particularly, to a cultivation support system, a cultivation support device, a cultivation support method, and a program for plants.

### BACKGROUND ART

As the aging of farmers has been a problem in recent years, larger-scale farming by companies etc., development of young people's need to be farmers, etc. are expected to increase people newly engaged in farming. Further, it has become common for individuals to grow plants for enjoyment (hereinafter, also referred to as gardening).

People newly engaged in farming and people doing gardening as a personal hobby as above often do not have sufficient knowledge and know-how about the cultivation of plants. Thus, techniques to support people who are not experts to cultivate plants have been proposed (e.g., Patent Document 1).

Patent Document 1 discloses a technique of measuring, nondestructively at low cost, indices representing the activity of a plant body, such as leaf surface temperature and water potential, by performing imaging processing from a point distant from the plant body with a combination of sensors (a CCD, an infrared sensor, and a bandpass filter), to allow a person who is not an expert to determine the condition of the plant. More specifically, it is presented not only to present images of a plant sensed as described above, but also to convert them into information that allows determination of whether variations in the leaf surface temperature of the plant are abnormal or not, based on saturation deficits obtained from the results of measurement of relative humidity, and present the information. It is also described to visually display differences from data such as leaf surface temperature and water potential on an ideal farmer (e.g., a farmer achieving a high yield), and with this, to display analysis on a user's cultivation method.

### PRIOR ART DOCUMENTS

### PATENT DOCUMENTS

Patent Document 1: Japanese Unexamined Patent Publication No. 2014-198012

### SUMMARY OF THE INVENTION

### PROBLEMS TO BE SOLVED BY THE INVENTION

The technique of Patent Document 1 allows a person who is not an expert to know the condition of a plant and to compare it with the condition of the plant of an ideal farmer. However, there is a problem that even if a person who does not have sufficient knowledge and know-how about the cultivation of a plant checks such data, the person does not know specifically when and what cultivation work should be performed to bring the plant closer to an ideal condition. Further, there are circumstances where the "ideal" condition of a plant varies depending on the purpose of or a plan for plant cultivation (hereinafter, collectively referred to as a cultivation plan).

The present invention has been made in view of the above circumstances, and an object thereof is to provide a technique that allows people who do not have sufficient knowledge and experience of plant cultivation to easily perform cultivation work required to achieve ideal plant cultivation.

### MEANS FOR SOLVING THE PROBLEM

In order to achieve the above object, the present invention adopts the following configuration.

A cultivation support system according to the present invention is a system for supporting cultivation of a plant, which includes:
a growth information acquisition unit configured to acquire information on a growing condition of the plant;
a growing condition evaluation unit configured to evaluate the growing condition of the plant on a scale of levels;
a work content determination unit configured to determine optimum cultivation work for the growing condition of the plant;
a work instruction information creation unit configured to create work instruction information indicating work to be performed by a person engaged in the cultivation of the plant, based on the cultivation work determined by the work content determination unit; and
an output unit configured to output the work instruction information created by the work instruction information creation unit.

Here, the person engaged in the cultivation of the plant is also referred to as the cultivator below. The "information on the growing condition of the plant (hereinafter, also referred to as the growth information)" includes the height of the plant, the stem diameter, the shape, color, and number of leaves, the size and number of fruits, the amount of photosynthesis, etc. The growth information may be automatically acquired based on sensing by permanent sensor devices, and may be acquired by manual input of the information by a user. In addition, for example, semiautomatic acquisition may be used in which individual growth information is acquired by analyzing an image of the plant arbitrarily taken by the user.

The "cultivation work" includes, but not limited to, for example, leaf cut, disbudding, thinning, flower thinning, irrigation, additional fertilization, support, agrochemical spraying, harvesting, adjustment of the surrounding environment, etc. The "work instruction information" can indicate the work content by various expressions, and may indicate the work content by words, a photograph, a diagram, or the like, or may be a display combining them. The information may be based on voice information presentation, or may be a combination of a voice and a graphical display, moving images, etc.

The above configuration allows the growing condition of the plant to be grasped on a scale of levels, and the optimum cultivation work to be presented according to the level of the growth of the plant. Consequently, even a person who does not have sufficient knowledge and experience of plant cultivation (hereinafter, also referred to as a cultivation beginner) can easily perform cultivation work required to achieve ideal plant cultivation.

The work instruction information may include a graph display showing the relationship between a lapse of time and the growing condition of the plant. Specifically, for example, a graph in which the growing condition of the plant is taken on the vertical axis, and a lapse of time is taken on the horizontal axis may be included in the work instruction information. The work instruction information including this display allows the person engaged in the cultivation of the plant (hereinafter, also referred to as the cultivator) to easily grasp changes in the growing condition in time series.

The growing condition evaluation unit may evaluate the growing condition of the plant at least from the viewpoint of nutritional growth. The growing condition evaluation unit may evaluate the growing condition of the plant from the viewpoints of nutritional growth and reproductive growth according to the kind of the plant.

For example, for foliage plants, leafy vegetables, etc., the growing condition of the plants may be evaluated based only on nutritional growth. On the other hand, for plants intended to harvest fruits, both the viewpoints of nutritional growth and reproductive growth are important. In this respect, having the above configuration allows evaluation of the growing condition of the plant by taking into account a balance between nutritional growth and reproductive growth, depending on the kind of the plant.

The work instruction information may include a graph showing the growing condition indicating the nutritional growth and the growing condition related to the reproductive growth in a matrix form, a display showing a current growing condition of the plant on the graph, and a display showing an attainment target of the cultivation of the plant on the graph.

This configuration allows a condition to be attained and a current growing condition of the plant to be visually checked, and thus can maintain and increase the motivation of the person engaged in work who sees the results of performing the work approaching the condition to be attained.

The cultivation support system may further include a cultivation plan acquisition unit configured to acquire a cultivation plan of the plant, in which the work content determination unit may determine the optimum cultivation work according to the cultivation plan acquired by the cultivation plan acquisition unit.

In plant cultivation, it is necessary to perform appropriate cultivation according to a plan such as to maximize the yield, advance the harvest time, or produce tasty ones (emphasize the sugar-acidity balance). That is, depending on a task to be prioritized, the growth level of the plant as a target point varies, or speed to reach the next growth level varies. It is necessary to perform cultivation work in response to this. It is difficult for a cultivation beginner to take action appropriate to such a plan. The above configuration allows even a cultivation beginner to easily perform cultivation work appropriate to a cultivation plan.

The cultivation support system may further include a work history acquisition unit configured to acquire information on a history of past cultivation work, in which the work content determination unit may determine the optimum cultivation work according to the past work history acquired by the work history acquisition unit.

Even when the growth level of the plant does not change (that is, it is positioned at the same growth level), cultivation work to be performed (or not to be performed) next may vary depending on what cultivation work has been performed in the past. In this regard, the above configuration, which can determine the optimum cultivation work by taking into account the past work history, can thus eliminate, for example, inconvenience that additional fertilization instruction information is presented many times.

The cultivation support system may further include an environmental information acquisition unit configured to acquire information on a growth environment of the plant, in which the work content determination unit may determine the optimum cultivation work according to the environmental information acquired by the environmental information acquisition unit.

Here, the information on the growth environment of the plant includes the temperature, humidity, illuminance, carbon dioxide (CO₂) concentration, soil moisture content, etc. of a place where the plant is cultivated. This configuration allows the instruction of cultivation work with the adjustment of the growth environment also taken into consideration, and is particularly suitable for cultivation in an indoor agricultural field.

The cultivation support system may further include a cultivator information acquisition unit configured to acquire information on the cultivation skill of the person engaged in the cultivation of the plant, in which the work content determination unit may determine the optimum cultivation work according to the information acquired by the cultivator information acquisition unit. The work instruction information creation unit may create the work instruction information according to the information acquired by the cultivator information acquisition unit.

Depending on differences in cultivators' experience, knowledge, and skill, the content of work that can be performed may vary, and the degree of understanding of instruction content may vary. In this regard, the above configuration allows the output of the work instruction information optimized in instructed work content, instruction expression, etc., according to the cultivation skill of the cultivator.

The invention of the application can also be regarded as a cultivation support device including the growth information acquisition unit, the growing condition evaluation unit, the work content determination unit, and the work instruction information creation unit.

A cultivation support method according to the present invention is a method for supporting the cultivation of a plant, which includes:
a growth information acquisition step of acquiring information on the growing condition of the plant;
a growing condition evaluation step of evaluating the growing condition of the plant on a scale of levels;
a work content determination step of determining optimum cultivation work for the growing condition of the plant;
a work instruction information creation step of creating work instruction information indicating work to be performed by a person engaged in the cultivation of the plant, based on the cultivation work determined in the work content determination step; and
an output step of outputting the work instruction information created in the work instruction information creation step.

The above steps may be executed at predetermined time intervals. A plant has individual differences, and a certain piece of cultivation work does not always produce the same results in all heads. It cannot be said that the reproducibility of effects for cultivation work is necessarily high. In this regard, by periodically executing the steps in this way, correction can be performed continually to lead to an optimum growing condition.

The present invention can also be regarded as a program for causing an information processing device to execute the above steps, and a computer-readable recording medium in which such a program is non-temporarily recorded.

The above configurations and processing steps can be combined with each other to constitute the present invention as long as no technical contradictions arise.

### EFFECT OF THE INVENTION

The present invention can provide the technique that allows people who do not have sufficient knowledge and experience of plant cultivation to easily perform cultivation work required to achieve ideal plant cultivation.

### BRIEF DESCRIPTION OF THE DRAWINGS

Fig. 1 is a schematic diagram illustrating a schematic configuration of a cultivation support system according to an application example of the present invention.
Fig. 2 is a flowchart illustrating a flow of part of processing performed in the cultivation support system according to the application example.
Fig. 3 is a schematic diagram illustrating a schematic configuration of a cultivation support system according to a first embodiment.
Fig. 4 is a first explanatory diagram illustrating an example of output of work instruction information according to the first embodiment.
Fig. 5A is a second explanatory diagram illustrating an example of output of the work instruction information according to the first embodiment. Fig. 5B is a third explanatory diagram illustrating an example of output of the work instruction information according to the first embodiment.
Fig. 6 is a flowchart illustrating a flow of part of processing performed in the cultivation support system according to the first embodiment.
Fig. 7 is a block diagram illustrating a configuration of a cultivation support system according to a second embodiment.
Fig. 8 is a schematic diagram illustrating a modification of the cultivation support system according to the second embodiment.

### MODE FOR CARRYING OUT THE INVENTION

Hereinafter, embodiments of the present invention will be described with reference to the drawings.

### <Application example>

### (Configuration of application example)

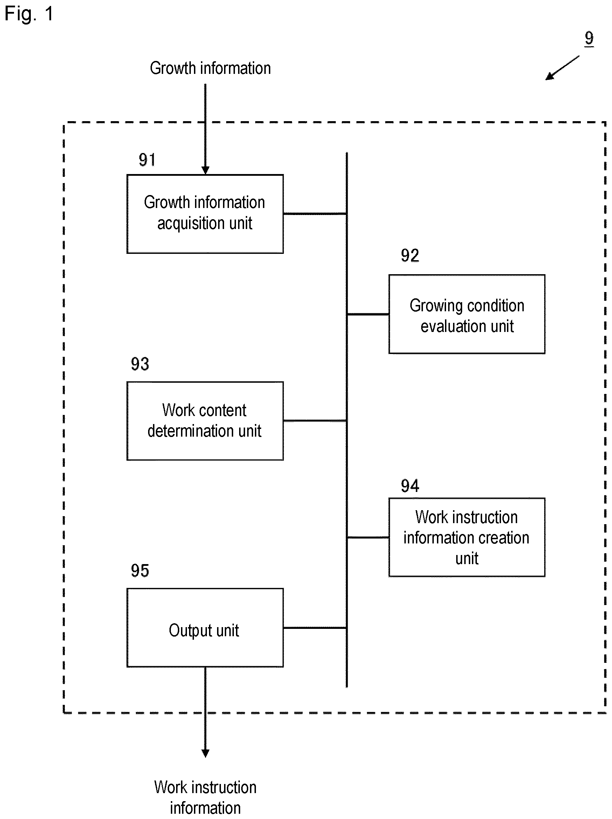

The present invention can be applied to, for example, a cultivation support device 9 as illustrated in Fig. 1. Fig. 1 is a block diagram illustrating a functional configuration of the cultivation support device 9 according to the application example. The cultivation support device 9 can be, for example, a general-purpose computer. Specifically, although not illustrated, an arithmetic processing unit, an input unit (such as a keyboard, a mouse, and a touch panel), an output unit (such as a liquid crystal display), and a storage unit (such as memory and a hard disk drive) are included. A communication unit (such as a LAN interface board and a wireless communication circuit) may also be included. Its form is not limited to a particular one. Various modes such as a desktop computer, a notebook computer, and a tablet information processing terminal are possible. A so-called smartphone may be used.

As illustrated in Fig. 1, the cultivation support device 9 includes functional units: a growth information acquisition unit 91, a growing condition evaluation unit 92, a work content determination unit 93, a work instruction information creation unit 94, and an output unit 95. These functional units are used to output information to support the cultivation work of a cultivator according to input information.

The growth information acquisition unit 91 acquires information on the growing condition of a plant via the input unit (including the communication unit). The information on the growing condition of the plant is, for example, information obtained from the appearance of the plant (such as the stem thickness and the number of fruits), information on photosynthesis obtained using a CO₂ sensor etc., and so on. For these pieces of information, information checked by a user may be input from the input unit, and information obtained from permanent sensor devices may be automatically input via the communication unit. In addition, for example, semiautomatic input may be used in which individual growth information is acquired by analyzing an image of the plant arbitrarily taken by the user.

The growing condition evaluation unit 92 evaluates the growing condition of the plant on a scale of levels, based on the information acquired by the growth information acquisition unit 91. Specifically, for example, for an index of nutritional growth, evaluation may be made on a scale of one to five (very high, high, proper, low, and very low). The evaluation is not limited to evaluation on a scale of one to five, and may be on a scale of one to a smaller number (e.g., three) or on a scale of one to a larger number (e.g., seven).

The work content determination unit 93 determines appropriate cultivation work according to the growth level of the plant evaluated by the growing condition evaluation unit 92. Here, the "cultivation work" includes, but not limited to, for example, leaf cut, disbudding, thinning, flower thinning, irrigation, additional fertilization, support, agrochemical spraying, harvesting, adjustment of the surrounding environment, etc. For the determination of cultivation work, for example, a table in which the growth levels of the plant are associated with the corresponding work may be prepared so that a determination is made by referring to the table according to the growth level.

The work instruction information creation unit 94 creates work instruction information indicating work to be performed by the cultivator, based on the work content determined by the work content determination unit 93. The work instruction information may be a text display showing the work content with words, or may be graphical content using a photograph, a drawing, or the like, or may be a display combining them. Alternatively, the information may be presented by voice or may be moving images.

The output unit 95 outputs the work instruction information via a liquid crystal display, a speaker, etc., depending on the content of the work instruction information.

### (Processing flow)

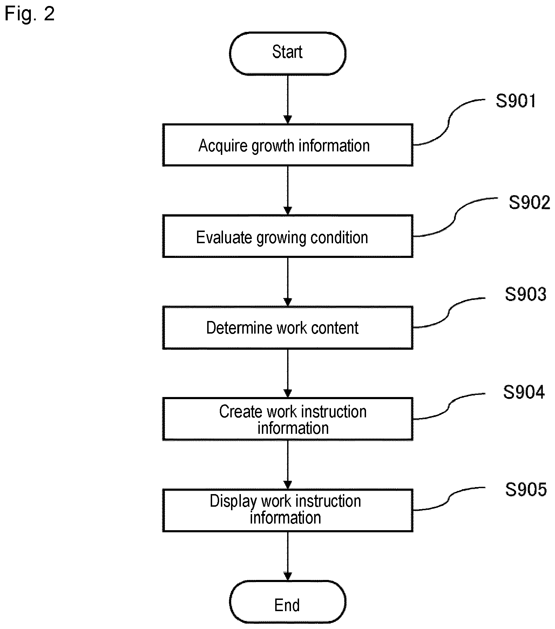

Next, with reference to Fig. 2, an example of processing to output work instruction information in the cultivation support device 9 having the above configuration will be described. Fig. 2 is a flowchart illustrating a flow of processing performed by the cultivation support device 9.

First, the cultivation support device 9 acquires growth information on a cultivated plant by the growth information acquisition unit 91 via the input unit and the communication unit of various types (S901). Next, based on the growth information acquired in step S901, the cultivation support device 9 evaluates the growing condition of the cultivated plant on a scale of levels by the growing condition evaluation unit 92 (S902). Further, the cultivation support device 9 determines work content by the work content determination unit 93 according to the growth level of the plant evaluated in step S902 (S903). Subsequently, the cultivation support device 9 creates work instruction information by the work instruction information creation unit 94, based on the work content determined in step S903 (S904), outputs the work instruction information (step S905), and completes a routine.

By using the cultivation support device 9 having the above configuration, even a cultivation beginner can easily perform cultivation work required to achieve ideal plant cultivation.

### <First embodiment

Next, a cultivation support system 1 that is another example of the mode for carrying out the invention will be described. However, the dimensions, materials, shapes, relative arrangements, etc. of components described in this embodiment are not intended to limit the scope of the invention only to them unless otherwise specified.

### (System configuration)

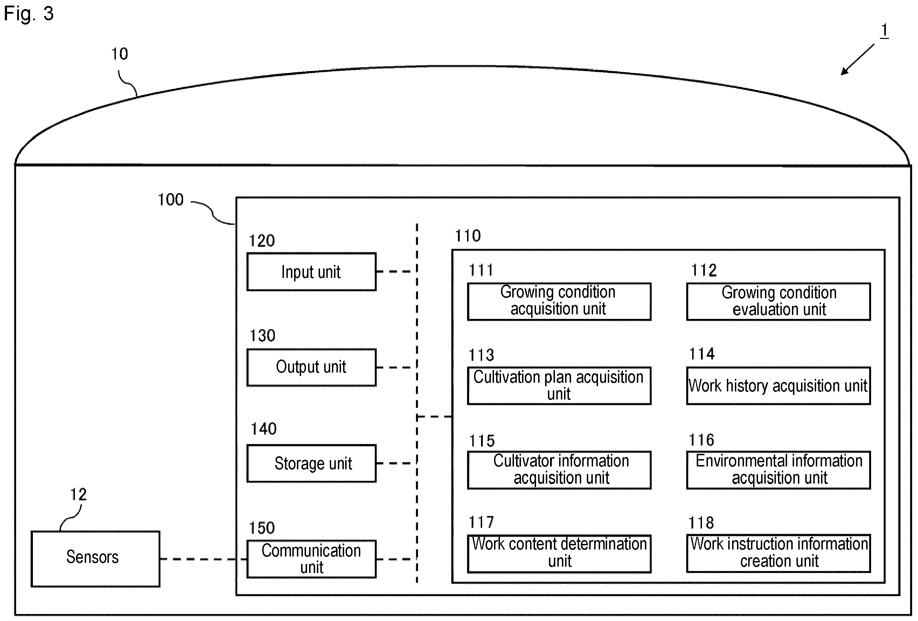

First, with reference to Fig. 3, an overall configuration of the cultivation support system 1 according to the embodiment will be described. Fig. 3 is a schematic diagram illustrating a system configuration of the cultivation support system 1. The cultivation support system 1 includes a greenhouse 10 for growing crops, a management terminal 100, a greenhouse environment adjustment mechanism 13, and various sensors 12.

The greenhouse 10 is an indoor agricultural field for cultivating plants therein, and corresponds to a so-called plastic greenhouse or the like, but is not limited to a particular material, size, etc., and includes those of various structures.

The management terminal 100 is a general computer. That is, the management terminal 100 is a computer that includes main memory such as read-only memory (ROM) or random-access memory (RAM), and an auxiliary memory such as an EPROM, a hard disk drive (HDD), or a removable medium. The management terminal 100 may be a single computer or may be composed of two or more computers that cooperate with each other.

The management terminal 100 is a general computer, and performs information communication with the greenhouse environment adjustment mechanism 13 and the various sensors 12, to control the environment in the greenhouse 10 and to provide a user with information on plant growth. Specifically, as illustrated in Fig. 3, a controller 110, an input unit 120 (such as a keyboard, a mouse, and a touch panel), an output unit 130 (such as a liquid crystal display and a speaker), a storage unit 140 (, such as a hard disk drive and flash memory), and a communication unit 150 (such as a LAN interface board and a wireless communication circuit) are included.

The controller 110 is a unit that controls the management terminal 100, and is a processor such as a central processing unit (CPU) or a digital signal processor (DSP). As functional modules related to cultivation support for a cultivator, a growing condition acquisition unit 111, a growing condition evaluation unit 112, a cultivation plan acquisition unit 113, a work history acquisition unit 114, a cultivator information acquisition unit 115, an environmental information acquisition unit 116, a work content determination unit 117, and a work instruction information creation unit 118 to be described later are included.

Although not illustrated, the storage unit 140 includes main memory such as read-only memory (ROM) or random-access memory (RAM), and an auxiliary memory such as an EPROM, a hard disk drive (HDD), or a removable medium. The auxiliary memory stores information to evaluate the growth level of the plant on a scale of levels (such as item-by-item threshold information), information to determine work content to be performed (such as a data table, an if-then rule, or a decision tree), an operating system (OS), various programs, etc., which will be described later. The stored programs are loaded into a work area of the main memory and executed, and the components and others are controlled through the execution of the programs, thereby being able to implement the functional units that accomplish predetermined purposes as described later.

The sensors 12 include at least a camera installed so as to be able to capture images of a plant cultivated in the greenhouse, and further include measuring devices to measure various physical quantities related to the environment in the greenhouse 10 (such as temperature, humidity, illuminance, and CO₂ concentration).

Next, the functional modules included in the controller 110 will be described. The growing condition acquisition unit 111 acquires data such as the stem thickness (stem diameter) of the plant, the leaf form at a growth point, the distance between the growth point and a flowering truss, and the number of fruits from image data acquired from the sensors 12.

Based on the information acquired by the growing condition acquisition unit 111, the growing condition evaluation unit 112 evaluates the growing condition of the plant on a scale of levels from the viewpoints of nutritional growth and reproductive growth. Specifically, for example, for the nutritional growth, thresholds to classify stem diameters into five levels are provided, and evaluation is performed based on them. For the reproductive growth, for example, thresholds to classify the numbers of fruits into five levels are provided, and evaluation is performed based on them.

The cultivation plan acquisition unit 113 acquires a cultivation plan desired by the cultivator. For example, it may perform processing to accept input of the plant cultivation plan (such as the maximum yield, the fastest harvest time, or emphasis on quality) via the input unit. It may store the cultivation plan input in this manner in the storage unit 140, and acquire the cultivation plan by reading information from there.

The work history acquisition unit 114 acquires a history of cultivation work performed in the past. For example, it may perform processing to accept input of the work history via the input unit, or may acquire the work history via the sensors 12. It may store the work history acquired in this manner in the storage unit 140 and read information from there to acquire the cultivation plan.

The cultivator information acquisition unit 115 acquires information on the cultivator's cultivation skill such as the cultivator's farming experience. For example, it may perform processing to accept input of the cultivator information via the input unit. Alternatively, it may read information on the work history stored in the storage unit 140 to acquire the cultivator information.

The environmental information acquisition unit 116 acquires information on the environment in which the plant is cultivated, such as the temperature, humidity, illuminance, carbon dioxide (CO₂) concentration, and soil moisture content in the greenhouse 10 via the sensors 12.

The work content determination unit 117 determines appropriate cultivation work to be performed by the cultivator, based on the growing condition of the plant evaluated by the growing condition evaluation unit 112. Specifically, for example, a table of plant growth levels and associated work may be stored in the storage unit 140 so that the work content determination unit 117 determines work content by referring to the table.

Multiple work content tables may be prepared according to the content of the cultivation plan, the past work history, the cultivator's cultivation skill, and the environmental information, to show work content in which these are reflected. For example, for the cultivation plan, work to be performed when it is desired to maximize the yield may be different from that when it is desired to emphasize quality (taste or sugar-acidity balance) even at the same growth level. To be able to respond to such differences, a level-by-level table may be prepared for each growth plan.

The work instruction information creation unit 118 creates work instruction information indicating work to be performed by the cultivator, based on the work content determined by the work content determination unit 117. The created work instruction information is output by the output unit. Its output mode may be words, a drawing, a photograph, a voice, moving images, a combination of them, etc., and is not limited to a particular one as long as it can be recognized by the cultivator. The work instruction information may be output together with other information.

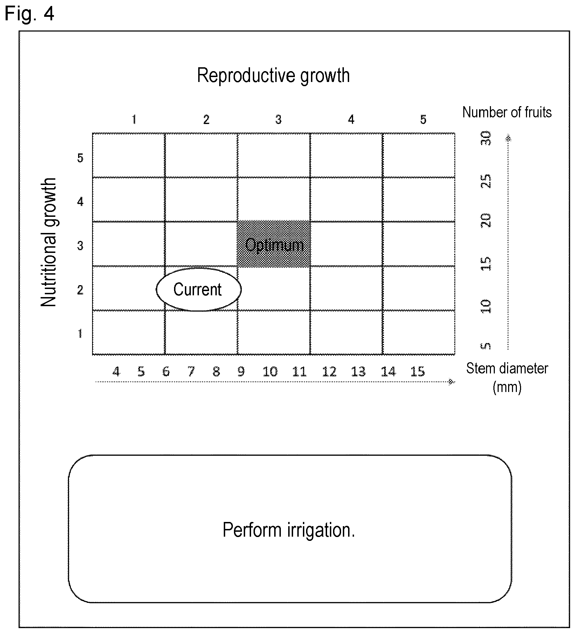

Fig. 4 illustrates an example of the work instruction information output on a liquid crystal display as the output unit. As illustrated in Fig. 4, the content of the work instruction is displayed as text, "Perform irrigation". The work instruction is displayed together with a graph showing the growing condition related to the nutritional growth and the growing condition related to the reproductive growth in a matrix form. Further, information indicating the current growing condition of the plant and information indicating a growing condition as a cultivation attainment target are displayed on the graph.

This graph display in addition to the cultivation work instruction allows the condition to be attained and the current growing condition of the plant to be visually checked, thus allowing the cultivator to maintain and increase motivation for the cultivation work.

The work instruction information may be expressed differently depending on the cultivation skill of the cultivator even when indicating the same work content.

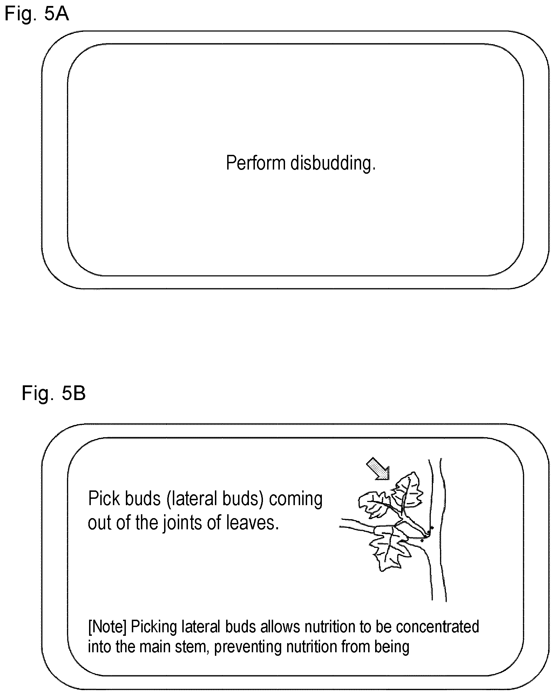

Figs. 5A and 5B illustrate examples of different expressions indicating the same work content (here, disbudding). Fig. 5A illustrates a display example for a cultivator having a certain level of cultivation skill, and Fig. 5B illustrates a display example for a cultivation beginner.

### (Processing flow)

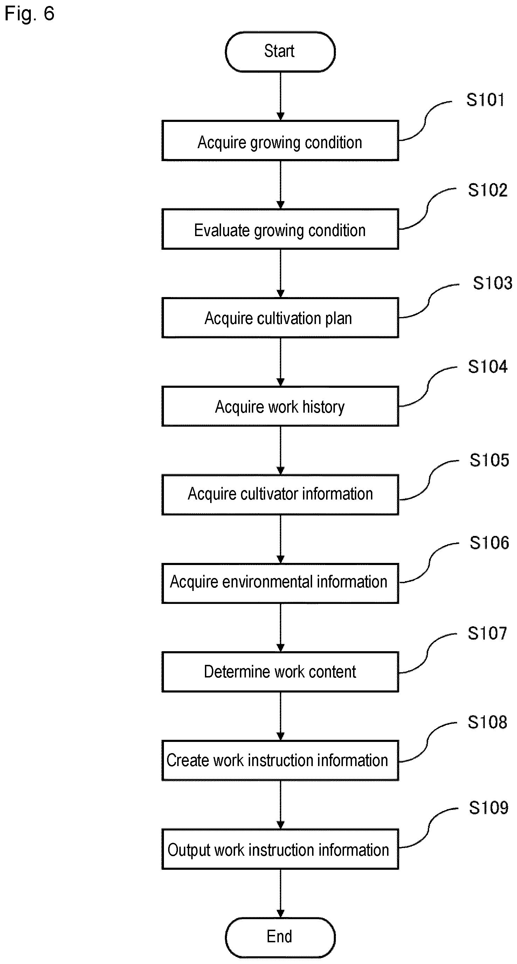

Next, with reference to Fig. 6, an example of processing to output work instruction information in the cultivation support system 1 having the above configuration will be described. Fig. 6 is a flowchart illustrating a flow of processing performed by the management terminal 100. The management terminal 100 performs the following processing using, for example, a work content output instruction by a cultivator via the input unit 120 as a trigger.

First, the management terminal 100 acquires information on the growing condition of a plant cultivated in the greenhouse 10 by the growing condition acquisition unit 111 (S101), and evaluates the growing condition of the plant by the growing condition evaluation unit 112, based on the growth information acquired in step S101 (S102).

Subsequently, the management terminal 100 acquires a cultivation plan from the cultivation plan acquisition unit 113 (S103), acquires a work history from the work history acquisition unit 114 (S104), acquires cultivator information from the cultivator information acquisition unit 115 (S105), and acquires environmental information from the environmental information acquisition unit S116 (S116).

Then, the management terminal 100 determines work content to be performed on the plant by the work content determination unit, based on the growing condition of the plant evaluated in step S102 and the information acquired in steps S103 to S106 (S107). Further, the management terminal 100 creates work instruction information for conveying the work content determined in step S107 to the cultivator by the work instruction information creation unit 118 (S108), outputs the work instruction information by the output unit 130 (S109), and completes a routine.

The routine does not necessarily need to be started with the cultivator's instruction as a trigger, and may be automatically performed at intervals of a predetermined time (such as one day, one week, or one month). The processing from step S103 to step S106 may be changed in order. The processing in steps S103 to S106 may be executed before steps S101 and S102.

The cultivation support system 1 having the above configuration can finely set work content to be performed, based on the cultivation plan for the plant, the history of work performed in the past, the cultivator's cultivation skill, and the growth environment of the plant in addition to the growing condition of the plant, to give more appropriate work instructions to the cultivator.

### <Second embodiment>

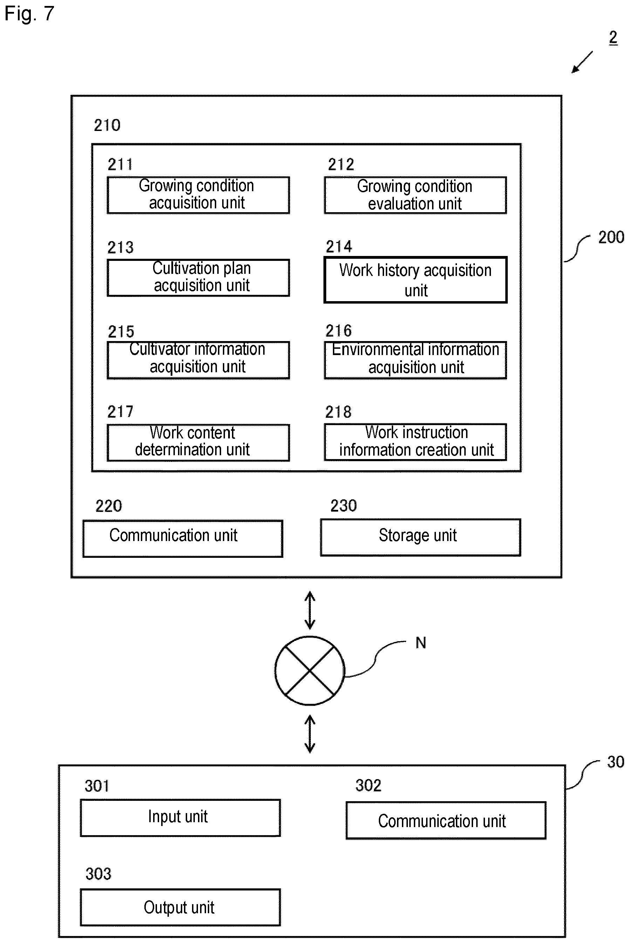

Subsequently, with reference to Fig. 7, an example of another embodiment of the present invention will be described. Fig. 7 is a block diagram illustrating an overall system configuration of a cultivation support system 2 according to the embodiment. As illustrated in Fig. 7, a center server 200 and an information processing terminal 30 are connected via a communication network N. As the communication network N, for example, a wide area network (WAN) that is a worldwide public communication network such as the Internet, or another communication network may be used. The communication network N may include a telephone communication network for mobile phones etc. and a wireless communication network such as Wi-Fi (registered trademark).

The center server 200 is a general server computer and includes a controller 210, a communication unit 220, and a storage unit 230.

The controller 210 is a unit that controls the center server 200, and is, for example, a CPU or the like. The controller 210 includes functional units: a growing condition acquisition unit 211, a growing condition evaluation unit 212, a cultivation plan acquisition unit 213, a work history acquisition unit 214, a cultivator information acquisition unit 215, an environmental information acquisition unit 216, a work content determination unit 217, and a work instruction information creation unit 218.

The communication unit 220 is a communication unit for connecting the center server 200 to the communication network N, and includes, for example, a LAN interface board and a wireless communication circuit for wireless communication.

The storage unit 230 is an EPROM, an HDD, or the like as described above, and stores an operating system (OS), various programs, various tables, and various data acquired via the communication network N.

The information processing terminal 30 is a general computer and includes an input unit 301, a communication unit 302, and an output unit 303. The information processing terminal 30 may be either a stationary terminal or a portable terminal. The information processing terminal 30 may be communicably connected to external devices such as sensors (not illustrated).

The input unit 301 is a unit to accept information input from the outside, such as a keyboard, a mouse, a touch panel, a camera, and a microphone. The communication unit 302 includes, for example, a LAN interface board and a wireless communication circuit for wireless communication. The output unit 303 includes a liquid crystal display, a speaker, etc.

The cultivation support system 2 according to the embodiment is different from that of the first embodiment in terms of information acquisition in the functional units of the growing condition acquisition unit 211, the cultivation plan acquisition unit 213, the work history acquisition unit 214, the cultivator information acquisition unit 215, and the environmental information acquisition unit 216.

Specifically, for example, the growing condition acquisition unit 211 acquires information on growing condition via the communication unit 220. That is, growth information is acquired by transmitting the growth information input from a cultivator via the input unit 301 of the information processing terminal 30 to the center server 200 via the communication network N.

Likewise, the other functional units of the cultivation plan acquisition unit 213, the work history acquisition unit 214, the cultivator information acquisition unit 215, and the environmental information acquisition unit 216 acquire information transmitted from the information processing terminal 30, or read information stored in the storage unit 230 after being transmitted to acquire necessary information.

The functions performed by the growing condition evaluation unit 212, the work content determination unit 217, and the work instruction information creation unit 218 are the same as those in the first embodiment and thus will not be described in detail.

In the cultivation support system 2 according to the embodiment, work instruction information created by the work instruction information creation unit 218 is transmitted from the communication unit 220 to the information processing terminal 30 via the communication network N, and is output from the output unit 303. That is, the cultivation support system 2 according to the embodiment is obtained by migrating part of the cultivation support system 1 according to the first embodiment to a cloud.

When a system operator installs the center server 200, the cultivation support system 2 having this configuration allows individual cultivators to easily perform cultivation work required to achieve ideal plant cultivation without preparing a dedicated terminal for cultivation support.

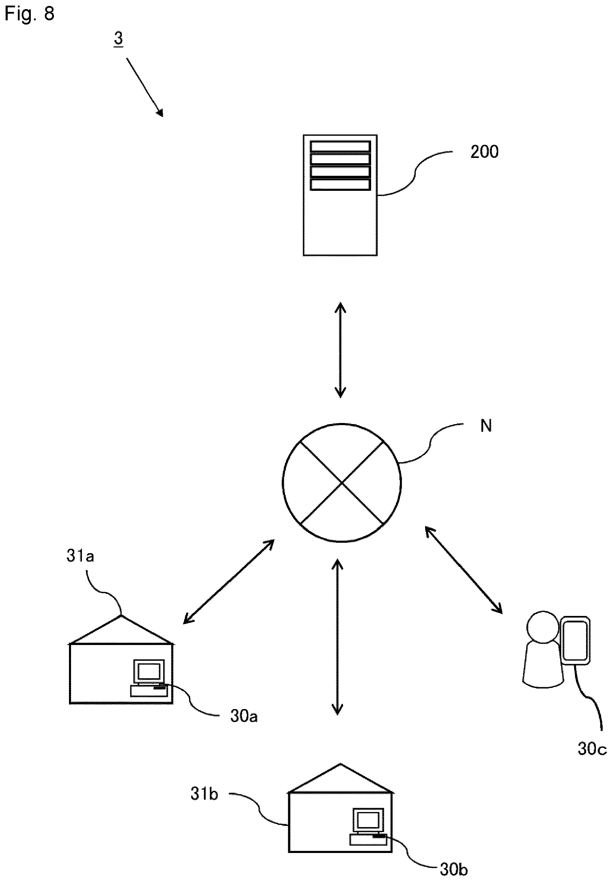

Fig. 8 illustrates a schematic diagram of a cultivation support system 3 that is a modification of the cultivation support system 2. As illustrated in Fig. 8, the cultivation support system 3 according to the embodiment has a configuration in which the center server 200 and a plurality of information processing terminals 30a, 30b, and 30c are connected via the communication network N.

The information processing terminals 30a and 30b in Fig. 8 are, for example, management terminals provided in farms 31a and 31b such as greenhouses, and the information processing terminal 30c is a portable information processing terminal such as a tablet terminal or a smartphone.

The information processing terminals 30 in the cultivation support system 3 may belong in the management of a plurality of different cultivators. Thus, by providing work instruction information to many cultivators, information such as growth information and environmental information acquired in the process can be collected and subjected to machine learning to improve the accuracy of work content determination.

### <Others>

The above embodiments are merely illustrative descriptions of the present invention, and the present invention is not limited to the above specific forms. The present invention allows various modifications and combinations within the scope of its technical idea. For example, the management terminal 100 and the center server 200 do not need to include all of the cultivation plan acquisition unit, the work history acquisition unit, the cultivator information acquisition unit, and the environmental information acquisition unit, and may not include part or all of them.

In the second embodiment, the sensors (not illustrated) may be provided with a communication function to transmit measured data directly to the center server 200, bypassing the information processing terminal 30.

The output examples of the work instruction information are merely an example, and it goes without saying that the displayed content and the display format can be variously modified. For example, when a leafy vegetable plant is cultivated, a graph in which the level of the nutritional growth of the plant is taken on the vertical axis, and a lapse of time is taken on the horizontal axis may be displayed. Of course, depending on the kind of the plant, the vertical axis may represent the level of the reproductive growth. The displayed content may be read aloud.

### <Supplementary note>

A mode of the present invention is
a cultivation support system (9, 1, or 2) for supporting cultivation of a plant, the system including:
a growth information acquisition unit (91, 111, or 211) configured to acquire information on the growing condition of the plant;
a growing condition evaluation unit (92, 112, or 212) configured to evaluate the growing condition of the plant on a scale of levels;
a work content determination unit (93, 117, or 217) configured to determine optimum cultivation work for the growing condition of the plant;
a work instruction information creation unit (94, 118, or 218) configured to create work instruction information indicating work to be performed by a person engaged in the cultivation of the plant, based on the cultivation work determined by the work content determination unit; and
an output unit (95, 130, or 303) configured to output the work instruction information created by the work instruction information creation unit.

A cultivation support method for supporting cultivation of a plant, the method including:
a growth information acquisition step (S901 or S101) of acquiring information on the growing condition of the plant;
a growing condition evaluation step (S902 or S102) of evaluating the growing condition of the plant on a scale of levels;
a work content determination step (S903 or S107) of determining optimum cultivation work for the growing condition of the plant;
a work instruction information creation step (S904 or S108) of creating work instruction information indicating work to be performed by a person engaged in the cultivation of the plant, based on the cultivation work determined in the work content determination step; and
an output step (S905 or S109) of outputting the work instruction information created in the work instruction information creation step.

### DESCRIPTION OF SYMBOLS

1, 2, 3, 9 cultivation support system
10 greenhouse
100 management terminal
110, 210 controller
200 center server
10, 31a, 31b greenhouse
30, 30a, 30b, 30c information processing terminal
N communication network

## Claims

1. A cultivation support system for supporting cultivation of a plant, the system comprising:
a growth information acquisition unit configured to acquire information on a growing condition of the plant;
a growing condition evaluation unit configured to evaluate the growing condition of the plant on a scale of levels;
a work content determination unit configured to determine optimum cultivation work for the growing condition of the plant;
a work instruction information creation unit configured to create work instruction information indicating work to be performed by a person engaged in the cultivation of the plant, based on the cultivation work determined by the work content determination unit; and
an output unit configured to output the work instruction information created by the work instruction information creation unit.

2. The cultivation support system according to claim 1, wherein
the growing condition evaluation unit evaluates the growing condition of the plant at least from a viewpoint of nutritional growth.

3. The cultivation support system according to claim 2, wherein
the growing condition evaluation unit evaluates the growing condition of the plant from viewpoints of nutritional growth and reproductive growth according to a kind of the plant.

4. The cultivation support system according to any one of claims 1 to 3, further comprising
a cultivation plan acquisition unit configured to acquire a cultivation plan of the plant,
wherein the work content determination unit determines the optimum cultivation work according to the cultivation plan acquired by the cultivation plan acquisition unit.

5. The cultivation support system according to any one of claims 1 to 4, further comprising
a work history acquisition unit configured to acquire information on a history of past cultivation work,
wherein the work content determination unit determines the optimum cultivation work according to the past work history acquired by the work history acquisition unit.

6. The cultivation support system according to any one of claims 1 to 5, further comprising
an environmental information acquisition unit configured to acquire information on a growth environment of the plant,
wherein the work content determination unit determines the optimum cultivation work according to the environmental information acquired by the environmental information acquisition unit.

7. The cultivation support system according to any one of claims 1 to 6, further comprising
a cultivator information acquisition unit configured to acquire information on cultivation skill of the person engaged in the cultivation of the plant.

8. The cultivation support system according to claim 7, wherein
the work content determination unit determines the optimum cultivation work according to the information acquired by the cultivator information acquisition unit.

9. The cultivation support system according to claim 7 or 8, wherein
the work instruction information creation unit creates the work instruction information according to the information acquired by the cultivator information acquisition unit.

10. The cultivation support system according to claim 1, wherein
the work instruction information includes a graph display showing a relationship between a lapse of time and the growing condition of the plant.

11. The cultivation support system according to claim 3, wherein
the work instruction information includes a graph showing the growing condition indicating the nutritional growth and the growing condition related to the reproductive growth in a matrix form, a display showing a current growing condition of the plant on the graph, and a display showing an attainment target of the cultivation of the plant on the graph.

12. A cultivation support device constituting at least part of the cultivation support system according to any one of claims 1 to 11, the cultivation support device comprising:
the growth information acquisition unit;
the growing condition evaluation unit;
the work content determination unit; and
the work instruction information creation unit.

13. A cultivation support method for supporting cultivation of a plant, comprising:
a growth information acquisition step of acquiring information on a growing condition of the plant;
a growing condition evaluation step of evaluating the growing condition of the plant on a scale of levels;
a work content determination step of determining optimum cultivation work for the growing condition of the plant;
a work instruction information creation step of creating work instruction information indicating work to be performed by a person engaged in the cultivation of the plant, based on the cultivation work determined in the work content determination step; and
an output step of outputting the work instruction information created in the work instruction information creation step.

14. The cultivation support method according to claim 13, wherein
the steps are executed at predetermined time intervals.

15. A program for causing an information processing terminal to execute the steps according to claim 13.
